Europäisches Patentamt

(19) European Patent Office

Office européen des brevets

(11) Publication number: **0 316 180**
**A1**

(12) # EUROPEAN PATENT APPLICATION

(21) Application number: **88310609.8**

(22) Date of filing: **10.11.88**

(51) Int. Cl.⁴: **A 61 B 5/04**

(30) Priority: **10.11.87 US 119355**

(43) Date of publication of application:
**17.05.89 Bulletin 89/20**

(84) Designated Contracting States:
**BE DE FR GB IT NL SE**

(71) Applicant: **Neward, Theodore Chester**
**c/o Neward Enterprises 9251 Archibald Avenue**
**Cucamonga California 91730 (US)**

(72) Inventor: **Neward, Theodore Chester**
**c/o Neward Enterprises 9251 Archibald Avenue**
**Cucamonga California 91730 (US)**

(74) Representative: **Barlow, Roy James et al**
**J.A.KEMP & CO. 14, South Square Gray's Inn**
**London WC1R 5EU (GB)**

(54) **Spring electrode.**

(57) An electrode (30) and carrier (50) assembly is disclosed for attachment to a biological subject, such as a fetus during labor to monitor the fetal heart rate. The electrode structure includes a clip-like body (26) having clamping portions and members (16a, 26a) adapted to engage cam surfaces within a carrier. The ends of the clamping portions which serve as electrodes are insulated (18) leaving only the tips exposed. While the electrode structure is housed within the carrier the clamping portions are held apart. Attachment is accomplished by placing the carrier (50) against the subject and moving the members into engagement with the cam surfaces (52a, 52b), whereby the cam surfaces eject the electrode structure from the carrier by the cam action thus allowing the clamping portions thereof to clamp into the subject's skin. The carrier may then be removed completely, leaving only the electrode structure with its associated wiring.

FIG. 2.

EP 0 316 180 A1

Description

## SPRING ELECTRODE

### BACKGROUND OF THE INVENTION

The field of the present invention relates to electrodes, and more particularly to an electrode and a carrier therefor suitable for attachment to a fetus during labor prior to delivery to monitor the fetal heart rate. It is desirable to monitor the fetal heart rate continuously during labor in order to obtain an immediate indication of the presence of undue distress or danger to the fetus as a result of reduced heart rate. Devices attached external to the mother's body have proven inadequate for this purpose because they do not clearly distinguish the fetal heart rate from that of the mother. Therefore, various devices have been developed for attachment directly to the fetus during labor. One such device is disclosed in Neward U.S. Patent No. 4,254,764 which discloses an electrode structure comprising a clip-like body having arms which support opposed clamping members which are preferably metal wires sharpened to a point so as to easily penetrate the fetal epidermis.

An important consideration in the design of a spring electrode is minimizing background noise. Background noise may be minimized through component materials selection, electrical connection techniques, electrode design and others. Heretofore, certain of the factors which contribute to the creation of background noise have not been completely known or understood, nor has their solution.

### SUMMARY OF THE INVENTION

According to the present invention, there is provided an electrode and carrier assembly particularly adapted for fetal monitoring. The end of the assembly is easily inserted into the birth canal during labor, is operated in a fashion familiar to doctors, attaches easily and safely to the fetus, requires a minimum of associated structure while attached, and may be easily detached from the fetus after delivery. The electrode structure includes a clip-like body supporting at least one electrode, which may be one of the clamping portions of the clip, and having members adapted to engage cammed surfaces within a carrier. The electrode structure is designed to minimize background noise by insulating the metal clamping portions leaving only the tips exposed.

### BRIEF DESCRIPTION OF THE DRAWINGS

Fig. 1 illustrates an electrode with an insulated tip connected to a lead wire;

Fig. 2 is a detailed side view, in cross section, of a portion of an electrode carrier with an electrode structure therein according to the present invention and partial cross section in a position suitable for storage prior to use;

Fig. 3 is a side view in cross section of a portion of an electrode carrier as in Fig. 2, showing the electrode structure as it is positioned after being partially ejected from the carrier for attachment to the fetal epidermis.

### DETAILED DESCRIPTION OF THE PREFERRED EMBODIMENT

The preferred embodiment of the present invention will now be described with reference to the drawings. For the convenience of description, any element identified by a numeral in one Figure will be represented by the same numeral in any other Figure.

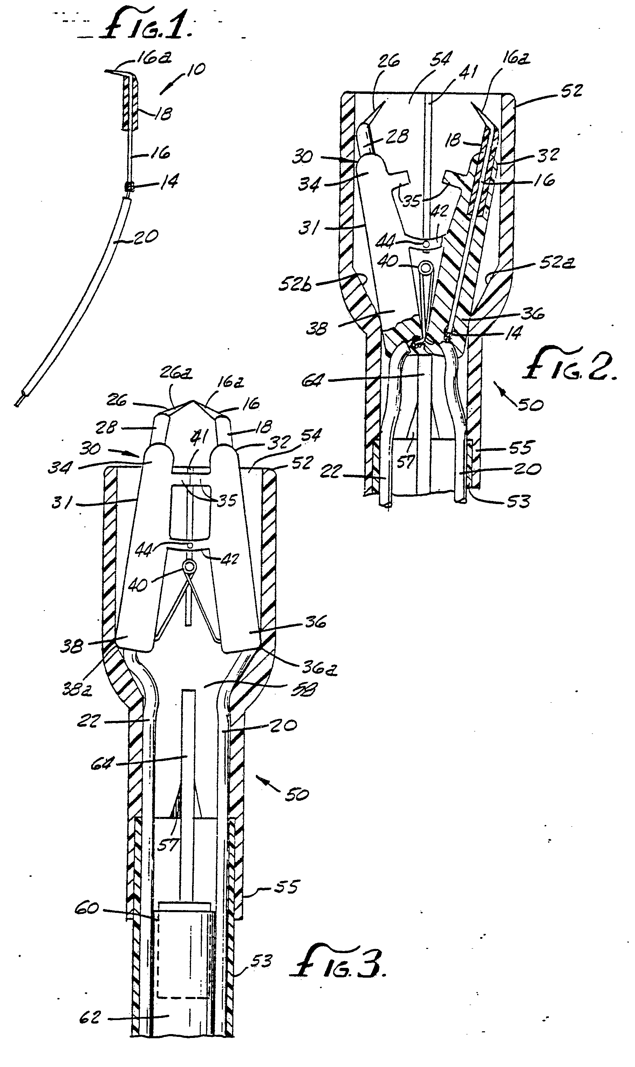

Fig. 1 illustrates an electrode 10 comprised of a metal wire 16 with a tip portion 16a having a bent and sharpened end. The end portion of wire 16 has an insulated portion 18 extending toward the end of tip 16a leaving only about 1.6 mm (1/16 of an inch) of metal exposed to make contact. The electrode wire 16 is then soldered at point 14 to a lead wire 20 leading to a terminal (not shown) which may be connected to an electronic fetal monitoring unit.

Fig. 2 illustrates the electrode 10 of Fig. 1 combined in an overall electrode structure 30 which is in the retracted position within a carrier 50. Fig. 3 illustrates the electrode 10 in the overall electrode structure 30 which is in the extended position out of the carrier 50. The electrode structure 30 and the carrier 50 are generally the same as disclosed in U.S. Patent No. 4,254,764 which is hereby incorporated by reference.

As viewed in Figs. 1 and 2 the electrode structure 30 is comprised of a clip type body 31 that may be constructed, for example, of a flexible plastic, having arms 32 and 34 supporting opposed clamping members 16 and 26 which are metal wires sharpened to a point to easily penetrate the fetal epidermis. The body 31 has legs 36 and 38 formed integrally with the arms 32 and 34, the pairs of arms and legs being connected by an integrally formed resilient web 35 so that the body 31 possess roughly the shape of an "H". A coil spring 40 is embedded in the legs 34 and 36 to exert an outward expansive force on the legs, with the result that the clamping members 16 and 26 are normally urged together or toward the closed position as in Fig. 3. The clamping member 16 in this embodiment serves also as a sensing electrode and is connected through the body 31 to the wire 20 which is ultimately connected to the monitoring unit. Another wire 22 leads from the body 31 and is connected within the body 31 to the spring 40 so that the spring 40 acts as a ground or reference electrode when the electrode structure 30 is surrounded by the maternal fluid present in the uterus prior to childbirth. A pair of guide pegs or pins 44 may be located on opposite sides of the web 42 of the body 31 for engagement with slots 41 in the carrier 50. Stops 35 may be provided on the facing surfaces of the arms 32 and 34 to allow the tips 16a and 26a to properly engage each other and to limit the effective range of expansion of the spring 40.

Two rounded protrusions (not shown) may be molded into the proximal end of the electrode housing 52. When the housing 52 is pressed against the subject, the protrusions gather a portion of the subject's skin into space between the protrusions to facilitate attachment to the skin by the clamping members 16 and 26. These protrusions are further described with reference to Fig. 8 in U.S. Patent No. 4,254,764 already incorporated.

The electrode carrier 50 includes an elongated flexible or curved cylinder or tube 53 and an electrode housing 52 joined by a cylindrical connecting member 55. The housing 52 and the connecting member 55 preferably comprise a single piece of molded plastic. The electrode housing 52 of the carrier 50 has an inner cavity 54 within which the electrode structure 30 is received. A raised portion 57 of the housing 52 provides a guide into a lower portion 58 of the cavity 54 into which the proximal end 64 of the plunger 60 is received as will later be described in more detail. A pair of slots 41 preferably are formed in the opposite inside walls of the housing 52 to receive the guide pins 44 of the electrode structure 30 and thereby insure proper alignment and simple attachment thereof. Cam surfaces 52a & b are formed in the lower part of the cavity 54 for engagement with the mating surfaces 36a and 38a of the respective legs 36 and 38 of the electrode body 31. The legs 36 and 38 are received within the connecting member 55 so that the clamping members 16 and 26 are separated while the electrode structure 30 is stored in the carrier 50.

Housed within the carrier 50 is a plunger 60 for moving the electrode structure 30 so that the mating surfaces 36a and 38a thereof move from their positions within the connecting member 55 onto the cam surfaces 52a and 52b for ejecting the electrode structure 30 from the carrier 50 and allowing the electrode structure 30 to attach to the subject. The plunger 60 comprises a flexible tube portion 62, part of which is shown in Fig. 3, and an end portion 64 having a flat, rectangular end adjacent to the electrode structure 30. The tube portion 62 is sufficiently smaller in diameter than the inside of the tube 53 to permit the electrode wires 20 and 22 to pass easily therethrough. When the plunger 60 is forced against the electrode structure 30 as in Fig. 2, the end portion 64 slides through the raised portion 57 of the housing 52 pushing out the electrode structure 30. The flat rectangular end of the end portion 64 allows steady, even pressure to be exerted against the electrode structure 30.

Once the electrode structure 30 is ejected and the clamping members 16 and 26 have attached, the carrier 50 may then slide over the wires 20 and 22 and be entirely removed. The wires 20 and 22 may then be reconnected to the monitoring unit.

The addition of the insulation 18 on the electrode 16 has shown unexpected and substantial reduction in background noise when monitoring fetal heart rate. The added insulation also strengthens the members 16 and 26. With the addition of the insulation 18, only about 1.6 mm(1/16 of an inch) of the tip 16a remains exposed. The exposed tip 16a may upon attachment enter into the skin leaving no metal or almost no metal of the clamping member 16 in contact with maternal fluid such that the tip 16a is completely or essentially embedded in the fetal skin. The insulation 18 may also serve as a stop preventing further insertion of the metal clamping member 16 into the skin.

In an alternate embodiment, the clamping member 26 may also be comprised of a wire and serve as a second electrode to contact with the fetal epidermis. In such an embodiment the tip 26a of the clamping member 26 is preferably provided with insulation 28 leaving only about 1.6 mm(1/16 of an inch) exposed similar to insulation 18 on tip 16a.

In order to manufacture an electrode 10 with the additional insulation 18 near the tip 16a, a completed device may be made in two stages. First, the wire 16 is bent and sharpened, and the insulation 18 is molded in a first die in order to produce the insulated shape of the clamping member electrode 16 as in Fig. 1. The first die supports the wire 16 at both ends during molding of the insulation 18. The electrode 16 is then soldered at point 14 to the lead wire 20. To complete the process, the electrode 10 is then held at end 16a in a second die to make the final molding for the electrode structure 30 (and particularly 32 and 36) as in Fig. 2 or 3. With the above method of manufacture, only 1.6 mm (1/16 of an inch) of metal is exposed at the tip 16a for making the electrical contact. This minimizing of exposed metal has been found necessary during testing to avoid unwanted background noise.

Thus, a spring electrode and method of manufacture are disclosed which reduces background noise during the monitoring process. While embodiments and applications of this invention have been shown and described, it would be apparent to those skilled in the art that other modifications are possible without departing from the inventive concepts herein. The invention, therefore, is not to be restricted accept as in the appended claims.

## Claims

1. Apparatus for use in physiological monitoring comprising

a clip-type spring electrode structure having a pair of legs, each of said legs supporting a clamping member, at least one of said members being of metal to serve as an electrode,

insulation on each clamping member which serves as an electrode leaving only a tip of the member exposed such that when the electrode is attached with the tip of the member penetrating into the subject's skin essentially none of the tip remains exposed,

means for connecting said metal electrode to monitoring equipment,

spring means acting upon said legs for urging said members together in a clamping fashion,

carrier means for housing said electrode structure prior to use with the clamping members separated, and

means for at least partially ejecting said electrode structure from said carrier means to allow said clamping members to be forced together and become attached to a subject when said carrier means is placed against such subject.

2. A fetal monitoring apparatus comprising a clip-type spring electrode structure having a pair of legs separated by a resilient web, each of said legs supporting a clamping member, at least one of said members being of metal to serve as an electrode,

insulation on each clamping member which serves as an electrode leaving only a tip of the member exposed, wherein substantially all of the exposed tip penetrates a subject's skin upon attachment of the electrode,

spring means acting upon said legs for urging said members together in a clamping fashion,

means for connecting said electrode to monitoring equipment,

an elongated tube having housing means at one end thereof for receiving said electrode structure, and said housing means including means for keeping said clamping members separated while said electrode structure is housed therein by engaging said legs and thereby compressing said spring means,

cam means within said housing means for engaging mating surfaces on said legs, and

a plunger extending through said tube for causing said mating surfaces to engage said cam means, whereby said electrode structure may be at least partially ejected from said housing means and said clamping members may be forced together and become attached to a subject when said housing means is placed against such subject.

3. A physiological monitoring electrode structure for association with a carrier having cam surfaces therein, said electrode structure comprising

a pair of opposed clamping members, at least one of said members being of metal to serve as an electrode,

insulation on each clamping member which serves as an electrode leaving only a tip of the member exposed, wherein substantially all of the exposed tip enters a subject's skin upon attachment of the electrode,

a pair of legs, each of said legs supporting a respective one of said members and said legs being separated by a spring which urges said members together, said legs being separated by a resilient web and having mating surfaces for engagement with said cam surfaces whereby said electrode structure may be at least partially ejected from said carrier when said mating surfaces engage with said cam surfaces, and

means for connecting said electrode to monitoring equipment.

4. Apparatus adapted for use in attaching a physiological monitoring electrode to the skin of a subject comprising

a substantially cylindrical tube,

a housing at one end of said tube adapted to receive a physiological monitoring electrode and adapted to be placed against the skin of the subject,

a plunger extending through said tube for exerting pressure on the electrode to eject the electrode from the housing,

a pair of protrusions extending from said housing for gathering the skin of a subject therebetween when said housing is placed against the subject to facilitate secure attachment of an electrode to the gathered skin when the electrode is ejected from the housing, and

insulation on each clamping member which serves as an electrode leaving only a tip of the member exposed, wherein substantially all of the exposed tip enters a subject's skin upon attachment of the electrode.

5. The apparatus of claim 4, wherein upon attachment of the electrode, the insulation touches the subject's skin.

6. A fetal monitoring apparatus comprising a clip-type spring electrode structure having a pair of legs separated by a resilient web, each of said legs supporting a clamping member, at least one of said members being of metal to serve as an electrode,

insulation on each clamping member which serves as an electrode leaving only a tip of the member exposed, wherein substantially all of the exposed tip enters a subject's skin upon attachment of the electrode,

spring means acting upon said legs for urging said members together in a clamping fashion,

means for connecting said electrode to monitoring equipment,

an elongated tube having housing means at one end thereof for receiving said electrode structure, and said housing means including means for keeping said clamping members separated while said electrode structure is housed therein by engaging said legs and thereby compressing said spring means,

cam means within said housing means for engaging mating surfaces on said legs,

a plunger extending through said tube for engaging said electrode structure to engage said cam means upon the application of pressure against said plunger, whereby said electrode structure may be at least partially ejected from said housing means and said clamping members may be forced together and thereby become attached to a subject when said housing means is placed against such subject, and

means mounted upon said housing means for gathering the skin of the subject against whom said housing means may be placed and thereby facilitating the secure attachment of said clamping members thereto.

7. A physiological monitoring electrode structure for association with a carrier having cam surfaces therein, said electrode structure comprising a pair of opposed sharpened wires mounted upon arm members, each arm member being connected to a respective leg member, said pairs of arm and leg members being joined by a resilient web, and said leg members being connected by a spring which urges said sharpened wires together, said leg members having mating surfaces for engagement with said cam surfaces whereby said electrode structure may be at least partially ejected from said carrier when said mating surfaces engage with said cam surfaces, and said electrode structure including means for connecting at least one of said sharpened wires to monitoring equipment, wherein each sharpened wire connected to monitoring equipment includes insulation thereon leaving only a tip of the wire exposed, wherein substantially all of the exposed tip enters a subject's skin upon attachment of the electrode.

8. The apparatus of any one of claims 1 to 7, further comprising means for halting penetration of the tip into the subject's skin, wherein the insulation comprises said means for halting penetration by touching the subject's skin.

9. A method for making an apparatus for physiological monitoring comprising the steps of:
   (a) bending a portion of a first end of a wire;
   (b) molding insulation onto the first end of the wire leaving only a tip of the wire exposed;
   (c) sharpening the tip of the wire;
   (d) connecting a second end of the wire to a lead wire; and
   (e) molding the wire into a clip-type spring electrode structure having a pair of legs which are movable in a clamping fashion, each of the legs supporting a clamping member, the wire being molded into one of the clamping members.

10. A method according to claim 19 further comprising the steps of:
   (f) installing a spring means for urging the clamping members together in the clamping fashion;
   (g) housing the electrode structure in a carrier means wherein prior to use, the clamping members are separated; and
   (h) providing a means for at least partially ejecting the electrode structure from the carrier means to allow the clamping members to be forced together and become attached to a subject when the carrier means is placed against the subject.

11. Apparatus according to any one of claims 1 to 8 or a method according to claim 9 or 10, wherein about 1.6 mm (1/16 inch) of the tip is left exposed from the insulation.

fig.1.

fig.2.

fig.3.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 007 702 (NEWARD)<br>* page 5, lines 25 - page 8, line 26; figures 1-3 * | 1-8 | A 61 B 5/04 |
| A | | 9 | |
| Y | US-A-3 580 242 (G.E. CROIX)<br>* abstract; column 2, lines 46 - 69; figures 1-4 * | 1-8 | |
| A | US-A-3 800 784 (J. KISZEL et al.)<br>* column 2, line 42 - column 3, line 12; figures 1-2 * | 1-4 | |

TECHNICAL FIELDS
SEARCHED (Int. Cl.4)

A 61 B 5/00

The present search report has been drawn up for all claims

| Place of search | Date of completion of the search | Examiner |
|---|---|---|
| BERLIN | 12-02-1989 | WEIHS J.A. |

EPO FORM 1503 03.82 (P0401)